# EUROPEAN PATENT SPECIFICATION

(11) **EP 4 385 400 B1**
(45) Date of publication and mention of the grant of the patent: **18.03.2026**
(21) Application number: 23214811.4
(22) Date of filing: 07.12.2023
(51) Int. Cl.: A61B 5/0205, A61B 5/08, A61B 5/251, A61B 5/00, A61B 5/0535

(54) **RESPIRATORY MEASUREMENT SYSTEM**
ATEMMESSSYSTEM
SYSTÈME DE MESURE RESPIRATOIRE

(30) Priority: 13.12.2022 US 202263432293 P; 01.12.2023 US 202318526514
(43) Date of publication of application: 19.06.2024
(73) Proprietor: Apple Inc., Cupertino, CA 95014 (US)
(72) Inventor: CHATTERJEE, Anirban, Cupertino, 95014 (US); KIM, Juseong, Cupertino, 95014 (US)
(74) Representative: Zacco Denmark A/S

(56) References cited:
- EP-A1- 3 431 004
- GB-A- 2 443 935
- US-A1- 2015 282 768

## Description

### FIELD

The described embodiments relate generally to systems for monitoring respiratory parameters of a user and more particularly to systems for determining respiratory rates for a user using impedance-based measurements.

### BACKGROUND

Wearable electronic devices are increasingly incorporating sensing systems that measure physiological parameters of a user. Depending on the physiological parameter being measured and the desired sensing modality used to make a measurement, certain sensing locations are more conducive to obtaining accurate and repeatable measurements. For example, a user's respiration rate may be more easily measured at a user's torso, which contracts and expands as the user breathes. Measuring respiration at a user's limb, such as at the wrist, or a user's head, may present challenges as movements of these portions of the body may be somewhat decoupled from movement of the torso during breathing. An exemplary wrist-worn device for measuring respiratory parameters of a user is disclosed in US 2015/282768 A1. It may be desirable to provide improved wearable electronic devices that are capable of measuring a user's respiration rate at various body locations.

### SUMMARY

The invention is defined by independent claims 1 and 8. Preferred embodiments are defined by their respective dependent claims.

### BRIEF DESCRIPTION OF THE DRAWINGS

The disclosure will be readily understood by the following detailed description in conjunction with the accompanying drawings, wherein like reference numerals designate like structural elements, and in which:
FIG. 1 shows a functional block diagram of an example electronic device that may be used to perform impedance-based respiratory measurements;
FIG. 2A shows an example watch that may incorporate a sensing system as described herein;
FIG. 2B shows a rear view of the example watch of FIG. 2A, which includes a sensing system positioned on the housing of the watch;
FIG. 3 shows an example process for performing impedance respiratory measurements using an electronic device;
FIG. 4 shows a visual example of impedance data generated by the sensing systems as described herein;
FIG. 5 shows an example process for selecting electrodes and performing impedance respiratory measurements using the selected electrodes as described herein;
FIG. 6 shows an example process for using motion data to determine when to perform impedance respiratory measurements; and
FIG. 7 shows a block diagram of an example electronic device that may incorporate a sensing assembly as described herein.

### DETAILED DESCRIPTION

Reference will now be made in detail to representative embodiments illustrated in the accompanying drawings. It should be understood that the following descriptions are not intended to limit the embodiments to one preferred embodiment. The invention is solely defined by the appended claims.

Embodiments disclosed herein are directed to devices and techniques for using impedance data to determine physiological parameters of a user. The devices can include one or more electrodes that are positioned to contact a user and are used to perform impedance measurements at a body part of a user. The devices can generate impedance data from the measurements and process the impedance data to determine physiological parameters including respiratory parameters, cardiac parameters, or other parameters of the user. In some cases, the impedance measurements may detect changes in blood flow through the body part of a user (e.g., as a result of the cardiac cycle) and the impedance data may be analyzed to identify shifts in the blood flow induced by the user's respiration. For example, the impedance measurements may capture pulsatile fluctuations of the blood flow and respiratory induced changes to the pulsatile blood flow. Analyzing the impedance data can include identifying the pulsatile characteristics of the blood flow and identifying respiratory induced shifts in the pulsatile signal. The devices and systems may derive one or more respiratory parameters, including a breathing rate, and display or otherwise output the parameter(s) to the user.

The impedance measurement systems described herein can be incorporated into a variety of electronic devices including wearable devices. Some wearable devices may be configured to be releasably coupled to a limb of a user (e.g., a user's arm or leg), and may include a smartwatch, bracelet, cuff, ring, band or the like. In some of these instances, the wearable device may be releasably secured to a user's wrist to allow the device to measure a user's respiratory parameters at the wrist. In other instances, example wearable devices may be configured to releasably couple to a user's head and may include a head-mounted device, headband, headphones, or the like. The wearable devices may be releasably secured to a user in any suitable manner, such as via a strap or other fastener (e.g., band of a smartwatch), via an adhesive (e.g., an adhesive patch), incorporated into clothing, or the like.

Electrodes can be positioned in a variety of configurations across one or more wearable devices. For example, in the case of a smartwatch, electrodes may be positioned on the body of the watch, a band of the watch, and/or a combination thereof. Additionally or alternatively, electrodes may be positioned on one or more other wearables such as an armband, headband, headphones, adhesive patch, or other wearable. In some instances, the wearable device performing impedance may not include a display that is used to present the respiration parameters to a user. In these cases, the wearable device may transmit impedance data (or the calculated respiration parameters) to an additional device having a display.

In some cases, the electronic devices may dynamically select a subset of the electrodes that is used to measure respiratory parameters. In this way, different subsets may be selected in different instances in an effort to increase the accuracy of these measurements. For example, the electronic device may perform a first set of measurements ("contact impedance measurements") to assess the quality of contact between different subsets of electrodes and the user's skin. The first set of measurements can be used to identify electrodes with sufficient (i.e., a threshold level) contact amount/quality with the user and select electrodes for performing a second set of measurements ("respiratory impedance measurements") based on the measured contact impedance. In some cases, the same electrodes can be operated as drive electrodes, which apply electrical signals to the user, and sense electrodes, which sense the applied electrical signals from the user. In other cases, different electrodes can be used as the drive and sense electrodes. For example, an impedance measurement can include applying an electric potential to the user via a pair of drive electrodes while measuring an impedance using a pair of sensing electrodes.

The electronic devices may use information from other sensors to determine when to initiate these measurements. For example, in some cases, the signal-to-noise ratio of respiratory impedance measurements may be improved by measuring the impedance during a low motion state of the user, which may thereby reduce noise associated with user movement. The electronic device can include a motion sensor that determines when a motion state of the device satisfies certain criteria (a low motion condition) that signifies that the electronic device is not moving in an undesirable manner. The electronic device may initiate and perform contact impedance measurements and/or respiratory impedance measurements while the electronic device satisfies the low motion condition. Accordingly, in some cases, the device can be configured to opportunistically obtain impedance data for the user when the movement is low, which may be while the user is sleeping, sitting, or otherwise in a low motion state.

In some cases, the impedance data can be combined with other data gathered from other sensors and analyzed together to determine one or more respiratory parameters of the user. For example, the impedance data can be analyzed with electrocardiogram (ECG) data, photoplethysmography (PPG) data, motion data, or any other suitable physiological data. The respiratory parameters may be determined in a variety of ways and/or from the multiple measurement sources. For example, the electronic device may collect a data set from each of a plurality of different sensor types. In some instances, the electronic device may only derive a respiration rate from one sensor type and its associated data set at any given time. For example, the physiological parameter may be derived from a sensing modality and its associated data set may be selected based on confidence metrics associated with each of the data sets. In other cases, the physiological parameter may be derived from a combination of the sensing modalities such as weighted average of respiration rates from two or more of the different data sets.

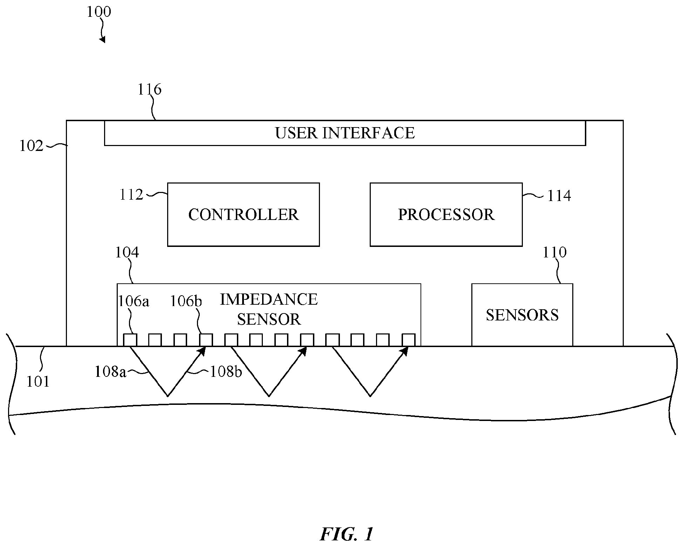

These and other embodiments are discussed below with reference to FIGs. 1-7. However, those skilled in the art will readily appreciate that the detailed description given herein with respect to these Figures is for explanatory purposes only and should not be construed as limiting.

FIG. 1 shows a functional block diagram of an example electronic device 100 that may be used to perform impedance-based respiratory measurements as described herein. The electronic device 100 can include an impedance sensor 104, which can include one or more electrodes 106 that contact a user 101. The electronic device 100 can also include other sensors 110, a controller 112, a processor 114, and a user interface 116. The electronic device 100 may include a housing 102 and one or more of the impedance sensor 104, the other sensors 110, the controller 112, and the processor 114, and the user interface may be positioned at least partially within the housing 102.

The impedance sensor 104 can include electrodes 106 that can be controlled to apply one or more electrical signals to the user 101 and/or sense the electrical signal after they have interacted with the user 101. In some cases, a first electrode 106a (or set of electrodes) can operate as a drive electrode, which applies one or more electrical signals 108a to the user 101 and a second electrode 106b (or set of electrodes) can be operated as a sense electrode, which measures the one or more electrical signals 108b that have been applied to the user 101. The processor 114 can determine one or more respiratory parameters based on impedance data generated by the controller 112 from the applied and sensed electrical signals 108. For example, the applied electrical signals 108a may be shifted or otherwise changed by blood flow through the body of the user 101. The controller 112 can generate the impedance data using the applied electrical signals 108a and the sensed electrical signals 108b, and the processor 114 can use the impedance data to determine a respiratory metric for a user, as described herein.

In some cases, the controller 112 can operate one or more electrodes 106 to function as drive and sense electrodes. In these cases, the same electrodes may apply the electrical signals 108a to the user and measure a return of the electrical signals 108b from the user 101. In other cases, the impedance sensor 104 may use one or more first electrodes 106a (one of which is labeled for clarity) as drive electrodes and one or more second electrodes 106b (one of which is labeled for clarity) as sense electrodes. The controller 112 can be configured to select the drive and sense electrodes 106. In some cases, the controller 112 can select the drive and sense electrodes 106 by analyzing electrical signals transmitted and/or sensed by the electrodes 106. For example, the controller 112 may perform contact impedance measurements for one or more of the electrodes 106, which may be used to determine electrodes that are used for additional respiratory impedance measurements to determine the physiological parameters of the user.

The impedance sensor 104 and/or controller 112 can include circuitry that connects different electrodes to the measurement circuitry for measuring impedance. For example, the impedance sensor 104 and/or controller 112 may include a multiplexor or other suitable electronic components that connect the selected electrodes to drive circuitry used to generate the applied electrical signals and sense circuitry used to measure impedance.

The electrical signals applied by the impedance sensor 104 may travel into the user's body via direct or indirect contact of the electrodes 106 with the user 101. The electrical signals may interact with the user 101, which may include a portion of the electrical signals being absorbed by the user's tissue (e.g., skin, blood, vessels, muscle, and so on) and/or modified by interaction with the user's tissue, and a portion of the electrical signals may be returned to the impedance sensor 104 where they can be sensed by one or more electrodes 106. The drive circuitry can apply a constant or time-varying voltage to tissue, which generates a current between the drive electrodes. The amount of current depends on the impedance of the tissue between the electrodes, and the drive circuitry measures this current to determine the impedance between the same electrodes. In some cases, the system can include dedicated sense electrodes and sense circuitry which measures the current to determined the impedance.

The sensed electrical signals may represent waveforms of the returned electrical signals 108b. The measured impedance may include a pulsatile component due to interactions with blood flow in the user 101 and/or a non-pulsatile component due to interactions with other tissue. The processor 114 may determine amplitudes of the pulsatile and/or non-pulsatile components of the sensed electrical signals, phase, modulation and/or the like, and determine the respiratory parameters based on the amplitudes, phase, modulation and so on.

The sensors 110 can include other types of sensors that are used to determine one or more parameters of a user. The sensors can include motion sensors such as an inertial measurement unit (IMU), light-based sensors such as PPG sensors, temperature sensors, ECG sensors, and/or any other suitable sensors. The sensors 110 can be operated in coordination with the impedance sensor 104. For example, the controller 112 and/or the processor 114 may use data from the sensors 110 to determine when to initiate impedance measurements. For example, a motion sensor may be used to identify a low motion state of the user 101, and the controller 112 may initiate impedance measurement at the impedance sensor 104 in response to identifying a low motion state of the user 101. In other cases, the controller 112 and/or processor 114 may use data from the sensors 110 to supplement the impedance data or as an alternative to the impedance data, as described herein.

The controller 112 may be operably coupled to the processor 114, the impedance sensors 104, and the other sensors 110. In some cases, all or part of the controller 112 may be implemented as computer-executable code executing on the processor 114. In some cases, all or part of the controller 112 may include or be implemented by dedicated hardware, including hardware for selectively operating electrodes in drive and/or sensor modes. In some cases, the controller 112 may include dedicated processing units for carrying out one or more functions of the impedance sensor 104 or other sensors 110 as described herein.

The user interface 116 may include a display, touch-sensitive display, speakers, microphones, haptic engines, or any other suitable input/output devices. The user interface may include components that are positioned within an interior of the electronic device 100, components that are partially within a housing of the electronic device 100 (e.g., a display) and/or components that are separate from and operably coupled to the electronic device 100 (e.g., headphones).

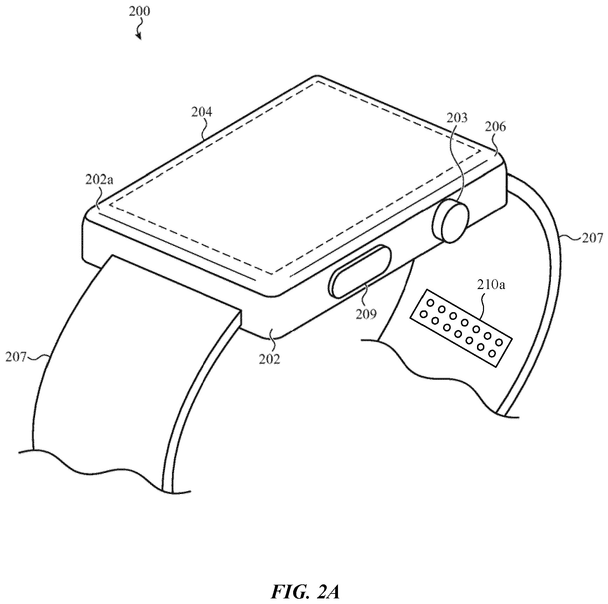

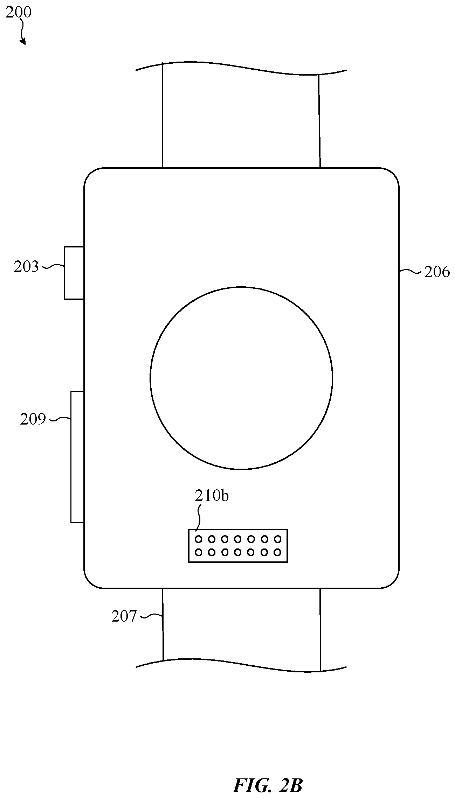

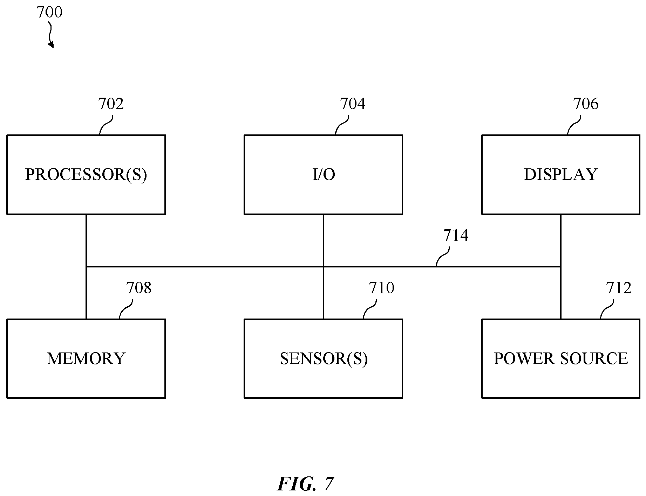

The electronic device 100 may include additional and/or alternative components that are not shown in FIG. 1. For example, the electronic device 100 may include input devices, output devices, memory, a power source, and/or electrical connectors that operably couple the components of the electronic device 100. Example components of the wearable electronic device 100 are discussed in more detail with respect to FIG. 7. Example electronic devices may include smartphones, tablets, portable music players, or other portable electronic devices; wearable electronic devices as described herein include a smartwatch (e.g., as described with reference to FIGs. 2A and 2B).

FIG. 2A shows an example watch 200 that may incorporate a sensing system as described herein. The watch 200 may be an electronic watch including a smartwatch that can incorporate the impedance sensing system and/or other sensing systems as described herein. The watch 200 may include a watch body 206 and a watch band 207. Other devices that may incorporate a crown assembly include other wearable electronic devices, other timekeeping devices, other health monitoring or fitness devices, other portable computing devices, mobile phones (including smart phones), tablet computing devices, digital media players, or the like. The watch 200 may have similar components, structure, and/or functionality as the wearable electronic device 100 described with respect to FIG. 1. The watch 200 may provide time and timing functions, receive messages and alerts, and may track activity of a user. In some cases, the watch may monitor biological conditions or characteristics of a user.

The watch body 206 may include a housing 202. The housing 202 may include a front side housing member that faces away from a user's skin when the watch 200 is worn by a user, and a back side housing member that faces toward the user's skin. Alternatively, the housing 202 may include a singular housing member, or more than two housing members. The one or more housing members may be metallic, plastic, ceramic, glass, or other types of housing members (or combinations of such materials).

The housing 202 may include a cover 202a mounted to form a front side of the watch body 206 (i.e., facing away from a user's skin) and may protect a display 204 mounted within the housing 202. The display 204 may produce graphical output that may be viewable by a user through the cover 202a. In some cases, the cover 202a may be part of a display stack, which may include a touch sensing or force sensing capability. The display may be configured to depict a graphical output of the watch 200, and a user may interact with the graphical output (e.g., using a finger, stylus, or other pointer). As one example, the user may select (or otherwise interact with) a graphic, icon, or the like presented on the display by touching or pressing (e.g., providing touch input) on the cover 202a at the location of the graphic. As used herein, the term "cover" may be used to refer to any transparent, semi-transparent, or translucent surface made out of glass, a crystalline material (such as sapphire or zirconia), plastic, or the like. Thus, it should be appreciated that the term "cover," as used herein, encompasses amorphous solids as well as crystalline solids. The cover 202a may form a part of the housing 202. In some examples, the cover 202a may be a sapphire cover. The cover 202a may also be formed of glass, plastic, or other materials.

The watch 200 may include one or more input devices (e.g., a crown 203, a button 209, a scroll wheel, a knob, a dial, or the like). The input devices may be used to provide inputs to the watch 200. The crown 203 and/or button 209 may be positioned along a portion of the housing 202, for example along a sidewall of the housing as shown in FIG. 2. In some cases, the housing 202 defines an opening through which a portion of the crown 203 and/or the button 209 extends.

The crown 203 may be user-rotatable, and may be manipulated (e.g., rotated, pressed) by a user. The crown 203 and/or button 209 may be mechanically, electrically, magnetically, and/or optically coupled to components within the housing 202, as one example. A user's manipulation of the crown 203 and/or button 209 may be used, in turn, to manipulate or select various elements displayed on the display, to adjust a volume of a speaker, to turn the watch 200 on or off, and so on.

In some embodiments, the button 209, the crown 203, scroll wheel, knob, dial, or the like may be touch-sensitive, conductive, and/or have a conductive surface, and a signal route may be provided between the conductive portion and a circuit within the watch body 206, such as a processing unit.

The housing 202 may include structures for attaching the watch band 207 to the watch body 206. In some cases, the structures may include elongate recesses or openings through which ends of the watch band 207 may be inserted and attached to the watch body 206. In other cases (not shown), the structures may include indents (e.g., dimples or depressions) in the housing 202, which may receive ends of spring pins that are attached to or threaded through ends of a watch band to attach the watch band to the watch body. The watch band 207 may be used to secure the watch 200 to a user, another device, a retaining mechanism, and so on.

In some examples, the watch 200 may lack any or all of the cover 202a, the display 204, the button 209, or the crown 203. For example, the watch 200 may include an audio input or output interface, a touch input interface, a force input or haptic output interface, or other input or output interface that does not require the display 204, the button 209, or the crown 203. The watch 200 may also include the aforementioned input or output interfaces in addition to the display 204, the button 209, or the crown 203. When the watch 200 lacks the display, the front side of the watch 200 may be covered by the cover 202a, or by a metallic or other type of housing member.

The watch 200 can include an impedance sensing system including an impedance sensor (e.g., impedance sensor 104) and controller (e.g., controller 112), as described herein. The impedance sensor can include one or more sets of electrodes positioned at different locations of the watch 200. For example, a first set of electrodes 210a may be positioned on the band 207 of the electronic device 100. In some cases, multiple sets of electrodes can be positioned at different locations on the band, although one set is shown for simplicity. In other cases, one or more sets of electrodes can include electrodes that are dispersed across a greater area of the band.

In some cases, the sensing systems described herein (e.g., impedance sensor 104, controller 112, other sensors 110, and the like) and other components of the electronic device (e.g., processor 114, user interface 116, and so on) can be incorporated into other wearable electronic devices. For example, a wearable electronic device may include a band that attaches to/wraps around the limb of a user and may not include watch components (e.g., housing, display, crown and/or other input buttons). For example, the band can include the sensing system components and/or other components integrated into the band structure.

In other cases, the sensing systems and/or other components can be integrated into other wearable devices such as head worn devices as described herein. The band, head worn device or other devices may or may not include a dedicated user interface. For example, a wearable device such as a band may be configured to communicate impedance measurements and/or impedance data to another device such as a smartphone, smartwatch, tablet, or computer that can output the impedance data and/or the determined physiological parameters to a user. In other cases, the wearable device may include a dedicated user interface that can display outputs, receive inputs, include speakers and/or microphones, include haptic device(s) and/or the like. These dedicated user interfaces may be configured to provide outputs and/or receive inputs primarily related to the physiological measurements (e.g., impedance measurements) being performed by the wearable device.

FIG. 2B illustrates a rear view of the example watch 200 of FIG. 2A. As shown in FIG. 2B, the watch body 206 defines a rear exterior surface of the watch 200. In some cases, one or more sets of electrodes may be positioned on the watch body 206. For example, a second set of electrodes 210b may be positioned along a back side of the watch body 206. In cases where different electrodes are positioned along different portions of the device (e.g., along the housing and the band), subsets of electrodes may be selected to include electrodes from the different portions of the device. The electrodes may be used to measure impedance of a user as described herein. The electrodes may be used to determine other biological parameters, such as a heart rate, an electrocardiogram, or the like. In some cases, the electrodes are used in combination with one or more additional electrodes, such as a surface of a crown assembly or other input device.

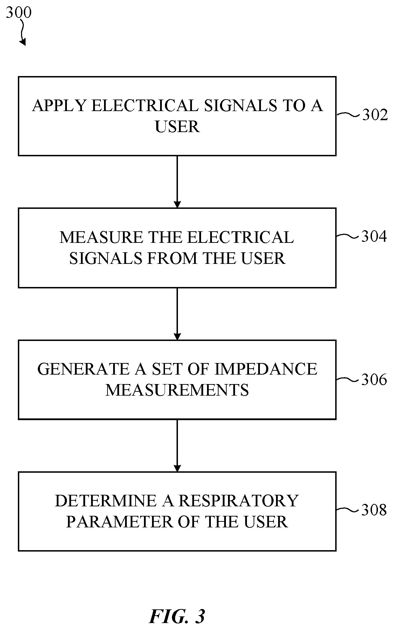

FIG. 3 shows an example process 300 for performing impedance respiratory measurements using an electronic device. The process 300 can be performed by the electronic devices described herein including electronic device 100 and watch 200.

At operation 302, the process 300 can include applying one or more electrical signals to a user. The electrical signals can be applied by one or more electrodes and applied at one or more locations of a user's body as described herein. For example, in some embodiments the electrical signals can be applied to a wrist of a user (e.g., via a watch) and in other embodiments the electrical signals can be applied to the head of the user (e.g., via a head mounted device such as a headband or earphones). The electrical signals can be applied as constant or time-varying voltage signals that generate currents in the user's skin at levels that are safe for the human body. The electrical signals can be applied by electrodes that are directly contacting the user's body (e.g., conductive surface of the electrode is placed directly on the skin of the user). In other cases, the electrodes may include a cover or other coupling mechanism that directly contacts the skin of the user. The cover or coupling mechanism can include conductive or semi-conductive materials, or other materials that help transmission of the electrical signals into the user's body.

At operation 304, the process 300 can include measuring the one or more electrical signals from the user. The electrical signals can be measured using the electrodes that transmitted the signals into the user and/or using different electrodes, as described herein. The electrical signals can be measured using electrodes that are directly contacting the user's body and/or using electrodes that include a cover or other coupling material between, as described herein.

Applying and sensing the electrical signals may be performed as a multiplexed operation in which applying the electrical signals and sensing the electrical signals are performed at different times. In other cases, the electrodes can be operated to apply and sense the electrical signals at the same time or during overlapping time periods.

At operation 306, the process 300 can include generating a set of impedance measurements from the applied and sensed electrical signals. The impedance data may include information about changes in the measured impedance over one or more time periods and may capture transient physiological processes such as pulsatile characteristics of blood flow through a user's body. The impedance data may be processed to convert the electrical measurements to digital signals. In some cases, the measured signals may be filtered (e.g., band-pass filtered), or otherwise processed to isolate specific effects such as changes in the measured impedance due to pulsatile blood flow in the user. Additionally or alternatively, the measured impedance data can be filtered or processed to remove noise and/or the like.

The impedance data may indicate specific sensor(s) that were used, a location of the sensors, indications of measurement quality (e.g., contact impedance), and/or other signal metrics. In some cases, the impedance data can span one or more time periods. For example, a first time period may capture a first series of blood pressure pulses in the user, which may span multiple respiration cycles. In some cases, a respiration cycle (inhalation and exhalation) may span multiple blood pressure pulses and the first time period may capture one or more respiration cycles. Additionally, the impedance data set may include one or more additional time periods that may overlap with or be discrete from the first time period. Each additional time period may capture one or more respiration cycles.

In some cases, each time period may be a predetermined period in which impedance data is collected. In some cases, each time period may correspond to one or more rolling windows. In other cases, the duration of the time period may be determined based on the measured impedance data and/or other physiological data collected by the electronic device. For example, the measurement data may be analyzed as it is collected and the duration of the time period may be determined based on the collected data meeting a defined confidence metric or other metric. In other cases, the duration may last a specified number of heart rate cycles and/or respiratory cycles, which may be determined from the impedance data and/or other measurement modalities, such as a motion sensor, ECG sensor, PPG sensor and so on.

At operation 308, the 300 process can include determining one or more respiratory parameters of the user from the impedance data. Determining respiratory parameters can include identifying respiratory induced changes across one or more blood pressure pulses. For example, the measurement engine and/or processing unit can be configured to identify blood pressure pulse amplitudes from the impedance data. Respiration may induce changes in the blood pressure pulse amplitudes across a respiration cycle. For example, the impedance may rise and fall across each blood pressure pulse, which may be used to identify a heart rate of a user. Additionally or alternatively, the average impedance amplitude of the blood pressure pulses may rise during inhalation and decrease during exhalation. In some cases, blood pressure pulses and impedance data may not be completely synchronized with the user's respiration, for example, the blood pressure pulses and impedance data may be phase shifted with respect to the respiration of the user. Accordingly, the device may be configured to identify rises in the average impedance value over multiple blood pressure pulses and/or phase shifts to identify a respiration rate of the user.

Additionally or alternatively, the impedance data can be used to determine other physiological parameters of a user. For example, the impedance data may be used to determine stroke volume variation based on changes in amplitudes of impedance signal, and/or respiratory sinus arrhythmia based on changes on heart rate changes between breaths.

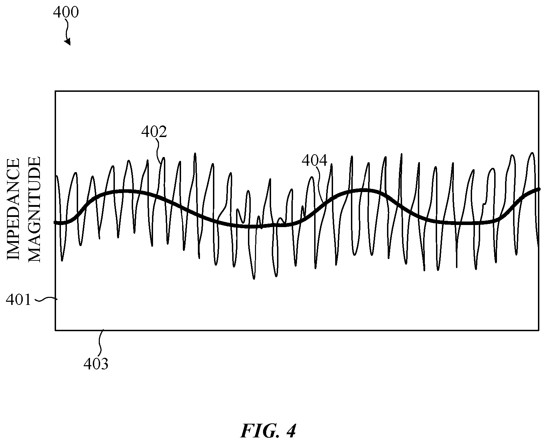

FIG. 4 shows a visual example of impedance data 400 generated by the sensing systems as described herein. The impedance data 400 is shown using a plot that includes a first axis 401 corresponding to the impedance magnitude and a second axis 403 corresponding to time. The impedance data 400 can include an impedance signal 402 that includes changes in the measured impedance over time. As shown in FIG. 4, the impedance signal may vary with heart rate and the changes in the impedance signal may track changes in each blood pressure pulse. For example, the changes in the impedance signal 402 may increase and decrease based on changes in blood volume and/or blood pressure within the user over each blood pressure pulse.

The impedance signal 402 can be used to identify a respiration rate of the user. In some cases, the amplitude of the impedance signal 402 will vary across one or more respiration cycles. For example, the respiration signal 404, which represents the change in impedance that is caused by respiration, can be determined from the impedance signal 402. This can include taking an average value of impedance amplitudes over each heart rate cycle, which can be used to generate the respiration signal 404. In other cases any other suitable techniques can be used to generate the respiration signal including curve fitting, rolling averages, the median value of each impedance cycle, and so on.

The respiration rate may be identified from the respiration signal 404, for example, from each peak of the respiration signal 404 (e.g., each peak represents a new breath). Additionally or alternatively, the respiration rate may be identified using other suitable techniques, which include identifying each valley of the respiration signal 404, averaging or otherwise analyzing multiple cycles of the respiration signal 404, and so on. In some cases, changes in the amplitude, average amplitude, phase, or other metric of the impedance signal 402 and/or the respiration signal may be used to determine a respiration rate and or other respiration metrics. For example, changes in amplitudes of the impedance signal may be used to determine a respiration depth, which may characterize how deeply a user is breathing (e.g., amount of inhalation and/or exhalation).

In some cases, the impedance signal 402 can be used to determined other physiological parameters of a user. For example, the pulse amplitude of each heart beat may change based on a stroke volume variation of the user. Accordingly, changes in the individual pulse amplitudes of the impedance signal 442 may be used to derive information about stroke volume variation of a user. Additionally or alternatively, heart rate may change between breaths, which may be indicated by frequency modulated changes in the impedance signal 402. Accordingly, changes in the frequency of the impedance signal 402 may be used to determine information about a user's respiratory sinus arrhythmia.

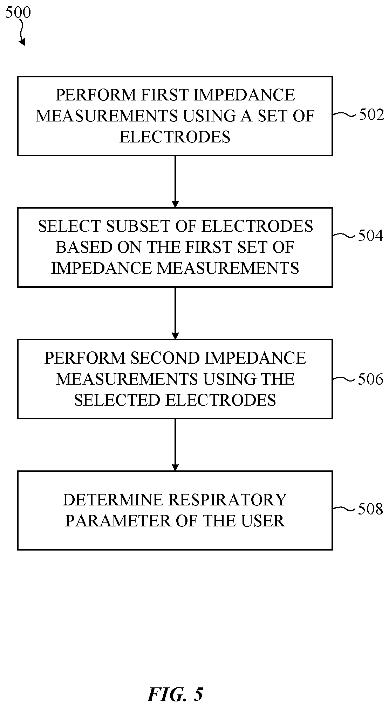

FIG. 5 shows an example process 500 for selecting electrodes and performing impedance respiratory measurements using the selected electrodes. The process 500 can be performed by the electronic devices described herein including electronic device 100 and watch 200.

At operation 502, the process 500 can include performing a first set of impedance measurements using one or more sets of electrodes. The first set of impedance measurements can include one or more contact metrics that characterize contact of electrodes with a user. For example, when a wearable device is worn by a user, the anatomy of a particular user and/or how the user is wearing the device can affect the contact that each of the electrodes has with the user. The first set of impedance measurements can be configured to apply electrical signals to the skin of the user to evaluate the ability of each electrode to transfer electrical signals to the user's tissue, such as blood vessels. The contact metric can be a contact metric of one or more electrodes with the user.

The contact metric can be determined for different combinations of electrodes within a set of electrodes. For example, if a set of electrodes includes three electrodes positioned on the back of a watch housing, the contact metric can be determined using different combinations of the three electrodes. In one case, the first electrode may apply an electrical signal to the user and a second electrode may sense the electrical signal from the user. Different combinations of electrodes can be evaluated to generate multiple skin contact impedance metrics that correspond to the different combinations of electrodes. Contact metrics can also be generated for electrodes in different sets, which may be located at different locations on the electronic device. For example, in the case of a watch, a set of electrodes may be located on a band of the watch. The system may determine contact metrics for different combinations of electrodes in the set of electrodes located on the band. Additionally or alternatively, the system may determine contact metrics for combinations of electrodes in different sets. For example, contact metrics for an electrode located on the watch body and an electrode on the watch band may be determined.

At operation 504, the process 500 can include selecting a subset of the electrodes based on the first set of impedance measurements. For example, the system may select a subset of electrodes with the lowest skin contact impedance. In some cases, the system may select the same electrodes to operate as drive and sense electrodes. In other cases, the system may select different electrodes to operate as drive electrodes and sense electrodes.

At operation 506, the process 500 can include performing second impedance measurements (e.g., respiratory impedance measurements as described herein) using the selected electrodes. The second impedance measurements can be used to generate impedance data for the user and determine one or more physiological parameters such as a respiratory rate as described herein.

In some cases, the system may periodically evaluate the contact of the selected subset of electrodes, for example, by performing additional skin contact impedance measurements. In some cases, the system may continue to use the selected subset of electrodes until the contact impedance metric drops below a contact criteria. In other cases, the system may use other sensors to determine when to evaluate the contact metric for the selected subset of electrodes and/or select different electrodes for performing the second impedance measurements. For example, the system may monitor motion of the user and, if a motion state of the user meets a motion threshold, the system may re-evaluate the contact metrics for the subset of electrodes and/or select a new subset of electrodes.

The system may also use the contact metrics to determine whether to perform the second impedance measurements. For example, the system may determine if the contact metrics for one or more combinations of electrodes satisfy an impedance threshold. If the system determines that the contact metrics satisfy the impedance threshold, the system may initiate the second set of impedance measurements. If the system determines that the contact metrics do not satisfy the impedance threshold, the system may abort or delay the second impedance measurement. In some cases, the system may continue to evaluate the contact metrics to determine if they satisfy the impedance threshold.

At operation 508, the process 500 can include determining a respiratory parameter of a user using impedance data generated from the selected subset of electrodes. Determining a respiratory parameter of the user can include identifying respiratory induced changes across one or more blood pressure pulses, as described herein.

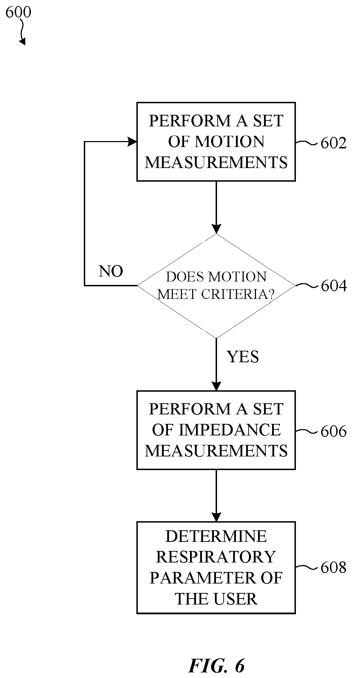

FIG. 6 shows an example process 600 for using motion data to determine when to perform impedance respiratory measurements. The process 600 can be performed by the electronic devices described herein including electronic device 100 and watch 200. The devices described herein may monitor for a low motion state prior to initiating impedance measurements of a user. In some cases, determining that a user is in a low motion state prior to initiating impedance measurements may help increase accuracy of the physiological parameters determined from the impedance data.

At operation 602, the process 600 can include performing a set of motion measurements of a user. The motion measurements can be used to determine a motion state of a user. In some cases, the motion measurements can be used to determine if the user is in a low motion state prior to initiating impedance measurements of the user. The motion measurements can be performed by one or more sensors including an IMU, such as one or more accelerometers which are configured to measure motion in one or more directions. The motion sensors may output an indication of the amount or magnitude of motion of the user. In some cases, outputs from the motion sensors may be used to determine a motion state of a user including whether a user is in a sleep state, rest state or the like.

The motion data may be analyzed in coordination with other data to determine a motion state of a user. For example, the motion data may be correlated to a time of day to determine whether a user is sleeping which may be associated with a longer duration low motion state. The impedance measurements may be adjusted accordingly, including collecting more impedance data sets during longer duration low motion states.

In some cases, the system may use impedance data obtained while the user is sleeping to generate a baseline data set of impedance based respiratory measurements. The system may opportunistically obtain respiratory impedance measurements of the user throughout the day, for example during shorter duration low motion states which may include while the user is sitting or otherwise sedentary. These opportunistic impedance measurements may be collected over a shorter duration and have higher thresholds for movements or other noise as compared to impedance measurements that are obtained while the user is sleeping. The opportunistic measurements may be compared or otherwise correlated to the baseline data set to evaluate a user's respiration changes from the baseline dataset.

At operation 604, the process 600 can include determining whether the motion measurements meet a low motion criteria. The low motion criteria may be defined to identify motion states in which the user is sleeping, resting, or in another low motion state. In some cases, the low motion criteria may be defined to identify when the movement of a user is below an acceleration threshold or other similar metric that characterizes the amount or intensity of movement of the user. The low motion criteria may include determining that a motion state of a user is below a threshold for a defined duration. For example, the low motion criteria may include a time duration that is used to differentiate between transient conditions and longer-term low motion conditions such as those associated with sleep periods or longer rest periods.

If the low motion state, determined from the motion measurements, does not meet the low motion criteria, the process 600 can include continuing to perform additional motion measurements of the user at operations steps 602 and 604 of process 600. In some cases, the process can include determining a wait time between performing a first set of motion measurements and a second set of motion measurements.

At operation 606, if the low motion state, determined from the motion measurements, meets the low motion criteria, the process 600 can include performing a set of impedance measurements for the user, as described herein. At operation 608, the process 600 can include determining one or more respiratory parameters of the user using the impedance data as described herein.

FIG. 7 is an example block diagram of an impedance measurement system 700, which can take the form of any of the devices as described with references to FIGs. 1-6. The impedance measurement system 700 can include a processor 702, an input/output (I/O) mechanism 704 (e.g., wired or wireless communications interfaces), a display 706, memory 708, sensors 710 (e.g., physiological sensors such as those described herein), and a power source 712 (e.g., a rechargeable battery). The processor 702 can control some or all of the operations of the impedance measurement system 700. The processor 702 can communicate, either directly or indirectly, with some or all of the components of the impedance measurement system 700. For example, a system bus or other communication mechanism 714 can provide communication between the processor 702, the I/O mechanism 704, the memory 708, the sensors 710, and the power source 712.

The processor 702 can be implemented as any electronic device capable of processing, receiving, or transmitting data or instructions. For example, the processor 702 can be a microprocessor, a central processing unit (CPU), an application-specific integrated circuit (ASIC), a digital signal processor (DSP), or combinations of such devices. As described herein, the term "processor" is meant to encompass a single processor or processing unit, multiple processors, multiple processing units, or other suitable computing element or elements. The processing unit can be programmed to perform the various aspects of the systems described herein.

It should be noted that the components of the impedance measurement system 700 can be controlled by multiple processors. For example, select components of the impedance measurement system 700 (e.g., a sensor 710) may be controlled by a first processor and other components of the impedance measurement system 700 (e.g., the I/O 704) may be controlled by a second processor, where the first and second processors may or may not be in communication with each other.

The I/O device 704 can transmit and/or receive data from a user or another electronic device. An I/O device can transmit electronic signals via a communications network, such as a wireless and/or wired network connection. Examples of wireless and wired network connections include, but are not limited to, cellular, Wi-Fi, Bluetooth, IR, and Ethernet connections. In some cases, the I/O device 704 can communicate with an external electronic device, such as a smartphone, electronic device, or other portable electronic device, as described here.

The impedance measurement system may optionally include a display 706 such as a liquid-crystal display (LCD), an organic light emitting diode (OLED) display, a light emitting diode (LED) display, or the like. If the display 706 is an LCD, the display 706 may also include a backlight component that can be controlled to provide variable levels of display brightness. If the display 706 is an OLED or LED type display, the brightness of the display 706 may be controlled by modifying the electrical signals that are provided to display elements. The display 706 may correspond to any of the displays shown or described herein.

The memory 708 can store electronic data that can be used by the impedance measurement system 700. For example, the memory 708 can store electrical data or content such as, for example, audio and video files, documents and applications, device settings and user preferences, timing signals, control signals, and data structures or databases. The memory 708 can be configured as any type of memory. By way of example only, the memory 708 can be implemented as random access memory, read-only memory, flash memory, removable memory, other types of storage elements, or combinations of such devices.

The impedance measurement system 700 may also include one or more sensors 710 positioned almost anywhere on the impedance measurement system 700. The sensor(s) 710 can be configured to sense one or more types of parameters, such as but not limited to, pressure, light, touch, heat, movement, relative motion, biometric data (e.g., biological parameters), and so on. For example, the sensor(s) 710 may include a pressure transducer, a heat sensor, an accelerometer, a gyroscope, a position sensor, a light or optical sensor, a magnetometer, a health monitoring sensor, and so on. Additionally, the one or more sensors 710 can utilize any suitable sensing technology, including, but not limited to, capacitive, ultrasonic, resistive, optical, ultrasound, piezoelectric, and thermal sensing technology.

The power source 712 can be implemented with any device capable of providing energy to the impedance measurement system 700. For example, the power source 712 may be one or more batteries or rechargeable batteries. Additionally or alternatively, the power source 712 can be a power connector or power cord that connects the impedance measurement system 700 to another power source, such as a wall outlet.

As described above, one aspect of the present technology is monitoring and managing physiological conditions of a user such as respiratory parameters and the like. The present disclosure contemplates that in some instances this gathered data may include personal information data that uniquely identifies or can be used to contact or locate a specific person. Such personal information data can include demographic data, location-based data, telephone numbers, email addresses, Twitter IDs (or other social media aliases or handles), home addresses, data or records relating to a user's health or level of fitness (e.g., vital signs measurements, medication information, exercise information), date of birth, or any other identifying or personal information.

The present disclosure recognizes that the use of such personal information data, in the present technology, can be used to the benefit of users. For example, the personal information data can be used to provide haptic or audiovisual outputs that are tailored to the user. Further, other uses for personal information data that benefit the user are also contemplated by the present disclosure. For instance, health and fitness data may be used to provide insights into a user's general wellness or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining the privacy and security of personal information data. Such policies should be easily accessible by users and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and revised to adhere to applicable laws and standards, including jurisdiction-specific considerations. For instance, in the US, collection of or access to certain health data may be governed by federal and/or state laws, such as the Health Insurance Portability and Accountability Act ("HIPAA"); whereas health data in other countries may be subject to other regulations and policies and should be handled accordingly. Hence different privacy practices should be maintained for different personal data types in each country.

Despite the foregoing, the present disclosure also contemplates embodiments in which users selectively block the use of, or access to, personal information data. That is, the present disclosure contemplates that hardware and/or software elements can be provided to prevent or block access to such personal information data. For example, in the case of determining spatial parameters, the present technology can be configured to allow users to select to "opt in" or "opt out" of participation in the collection of personal information data during registration for services or anytime thereafter. In addition to providing "opt in" and "opt out" options, the present disclosure contemplates providing notifications relating to the access or use of personal information. For instance, a user may be notified upon downloading an app that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of personal information data to implement one or more various disclosed embodiments, the present disclosure also contemplates that the various embodiments can also be implemented without the need for accessing such personal information data. That is, the various embodiments of the present technology are not rendered inoperable due to the lack of all or a portion of such personal information data. For example, haptic outputs may be provided based on non-personal information data or a bare minimum amount of personal information, such as events or states at the device associated with a user, other non-personal information, or publicly available information.

The foregoing description, for purpose of explanation, has been described with reference to specific embodiments. However, the illustrative discussions above are not intended to be exhaustive or to limit the invention to the precise forms disclosed. Many modifications are possible in view of the above teachings, as far as they are encompassed by the appended claims.

The foregoing description, for purposes of explanation, used specific nomenclature to provide a thorough understanding of the described embodiments. However, it will be apparent to one skilled in the art that the specific details are not required in order to practice the described embodiments. Thus, the foregoing descriptions of the specific embodiments described herein are presented for purposes of illustration and description. They are not targeted to be exhaustive or to limit the embodiments to the precise forms disclosed. It will be apparent to one of ordinary skill in the art that many modifications are possible in view of the above teachings, as far as they are encompassed by the appended claims.

## Claims

1. A wearable device (100, 200) comprising:
a band (207) configured to secure the wearable device to a wrist of a user;
a plurality of electrodes (106a, 106b, 210a, 210b) positioned to contact the user when the band secures the wearable device to the wrist of the user;
a motion sensor (110) positioned at least partially within the wearable device;
a processor (114) configured to:
perform a first set of measurements using the plurality of electrodes to determine first contact metrics for a plurality of subsets of the plurality of electrodes;
select a first subset of the plurality of subsets using the first contact metrics;
cause the plurality of electrodes to apply electrical signals to the user;
measure the electrical signals from the user using the selected first subset of the plurality of electrodes;
generate a set of impedance data from the measured electrical signals;
identify one or more respiratory cycles using the set of impedance data;
determine a motion state of the user using the motion sensor; and
in response to the motion state satisfying a first motion criteria, perform a second set of measurements using the plurality of electrodes to determine second contact metrics for a plurality of subsets of the plurality of electrodes;
select a second subset of the plurality of subsets using the second contact metrics; and
use the selected second subset of the plurality of subsets to measure electrical signals from the user.

2. The wearable device of claim 1, wherein each contact metric of the plurality of contact metrics represents a contact quality of a corresponding subset of the plurality of subsets.

3. The wearable device of any of claims 1-2, wherein the processor is configured to:
determine a first subset of electrodes from the plurality of electrodes that is used to apply the electrical signals to the user; and
determine a second subset of electrodes from the plurality of electrodes that is used to measure the electrical signals from the user.

4. The wearable device of claim 3, wherein the first subset of electrodes comprises different electrodes from the second subset of electrodes.

5. The wearable device of any of claims 1-4, wherein the processor is configured to measure electrical signals of the user in response to determining that the motion state satisfies a low motion criteria.

6. The wearable device of any of claims 1-5, further comprising an optical sensor coupled to the wearable device, wherein the processor is configured to:
generate a set of optical data using the optical sensor; and
identify the one or more respiratory cycles using the set of optical data.

7. The wearable device of any of claims 1-6, further comprising:
a housing; and
a touch-sensitive display coupled to the housing; wherein:
the band is configured to secure the housing to the wrist of a user; and
at least one of the plurality of electrodes is positioned on the band.

8. A wearable device (100, 200) comprising:
electrodes (106a, 106b, 210a, 210b) configured to:
apply one or more electrical signals to a user; and
sense the one or more electrical signals from the user;
a motion sensor (110) positioned at least partially within the wearable device; and
a processor (114) configured to:
perform one or more first impedance measurements using the electrodes to determine a first set of contact metrics;
select a first set of the electrodes using the first set of contact metrics;
perform one or more second impedance measurements using the first set of the electrodes to generate a first set of impedance data;
determine one or more respiratory cycles of the user from the set of impedance data;
determine a motion state of the user using the motion sensor;
in response to the motion state satisfying a motion criteria, perform one or more third impedance measurement using the electrodes to determine a second set of contact metrics;
select a second set of the electrodes using the second set of contact metrics: and
perform one or more fourth impedance measurements using the second set of electrodes to generate a second set of impedance data.

9. The wearable device of claim 8, wherein determining the first and second sets of contact metrics comprises determining a contact impedance for one or more of the electrodes.

10. The wearable device of any of claims 8-9, wherein the selecting the first and second sets of electrodes comprises:
selecting a first subset of the electrodes for applying the one or more electrical signals to the user; and
selecting a second subset of the electrodes for sensing the one or more electrical signals from the user.

11. The wearable device of claim 10, wherein the first subset of the electrodes comprises different electrodes from the second subset of the electrodes.

12. The wearable device of any of claims 8-11, wherein the processor is configured to:
determine if the first set of contact metrics satisfies an impedance threshold; and
in response to determining that the set of contact metrics satisfies the impedance threshold, initiating the one or more second impedance measurements.

13. The wearable device of claim 12, wherein in response to determining that the first set of contact metrics does not satisfy the impedance threshold, the processor is configured to abort the one or more second impedance measurements.

14. The wearable device of any of claims 8-13, wherein:
the electrodes comprise a first group of electrodes positioned at a first location of the wearable device and a second group of electrodes positioned at a second location of the wearable device; and
the selecting the first set of the electrodes comprises selecting one of the first set of electrodes or the second set of electrodes.

15. The wearable device of claim 14, further comprising:
a housing; and
a band configured to secure the housing to the user, wherein the first set of electrodes is positioned on the band.

## Patentansprüche

1. Am Körper tragbare Vorrichtung (100, 200), umfassend:
ein Band (207), das dazu konfiguriert ist, die am Körper tragbare Vorrichtung an einem Handgelenk eines Benutzers zu sichern;
eine Vielzahl von Elektroden (106a, 106b, 210a, 210b), die dazu positioniert ist, den Benutzer zu kontaktieren, wenn das Band die am Körper tragbare Vorrichtung an dem Handgelenk des Benutzers sichert;
einen Bewegungssensor (110), der mindestens teilweise innerhalb der am Körper tragbaren Vorrichtung positioniert ist;
einen Prozessor (114), der zu Folgendem konfiguriert ist:
Durchführen eines ersten Satzes von Messungen unter Verwendung der Vielzahl von Elektroden, um erste Kontaktmetriken für eine Vielzahl von Teilsätzen der Vielzahl von Elektroden zu bestimmen;
Auswählen eines ersten Teilsatzes der Vielzahl von Teilsätzen unter Verwendung der ersten Kontaktmetriken;
Veranlassen der Vielzahl von Elektroden, elektrische Signale auf den Benutzer anzuwenden;
Messen der elektrischen Signale von dem Benutzer unter Verwendung des ausgewählten ersten Teilsatzes der Vielzahl von Elektroden;
Erzeugen eines Satzes von Impedanzdaten aus den gemessenen elektrischen Signalen;
Identifizieren eines oder mehrerer Atmungszyklen unter Verwendung des Satzes von Impedanzdaten;
Bestimmen eines Bewegungszustands des Benutzers unter Verwendung des Bewegungssensors; und
als Reaktion darauf, dass der Bewegungszustand ein erstes Bewegungskriterium erfüllt, Durchführen eines zweiten Satzes von Messungen unter Verwendung der Vielzahl von Elektroden, um zweite Kontaktmetriken für eine Vielzahl von Teilsätzen der Vielzahl von Elektroden zu bestimmen;
Auswählen eines zweiten Teilsatzes der Vielzahl von Teilsätzen unter Verwendung der zweiten Kontaktmetriken; und
Verwenden des ausgewählten zweiten Teilsatzes der Vielzahl von Teilsätzen, um elektrische Signale von dem Benutzer zu messen.

2. Am Körper tragbare Vorrichtung nach Anspruch 1, wobei jede Kontaktmetrik der Vielzahl von Kontaktmetriken eine Kontaktqualität eines entsprechenden Teilsatzes der Vielzahl von Teilsätzen darstellt.

3. Am Körper tragbare Vorrichtung nach einem der Ansprüche 1-2, wobei der Prozessor zu Folgendem konfiguriert ist:
Bestimmen eines ersten Teilsatzes von Elektroden aus der Vielzahl von Elektroden, der dazu verwendet wird, die elektrischen Signale auf den Benutzer anzuwenden; und
Bestimmen eines zweiten Teilsatzes von Elektroden aus der Vielzahl von Elektroden, der dazu verwendet wird, die elektrischen Signale von dem Benutzer zu messen.

4. Am Körper tragbare Vorrichtung nach Anspruch 3, wobei der erste Teilsatz von Elektroden unterschiedliche Elektroden aus dem zweiten Teilsatz von Elektroden umfasst.

5. Am Körper tragbare Vorrichtung nach einem der Ansprüche 1-4, wobei der Prozessor dazu konfiguriert ist, elektrische Signale des Benutzers als Reaktion darauf zu messen, dass der Bewegungszustand ein Niedrigbewegungskriterium erfüllt.

6. Am Körper tragbare Vorrichtung nach einem der Ansprüche 1-5, umfassend einen optischen Sensor, der mit der am Körper tragbaren Vorrichtung gekoppelt ist, wobei der Prozessor zu Folgendem konfiguriert ist:
Erzeugen eines Satzes optischer Daten unter Verwendung des optischen Sensors; und
Identifizieren des einen oder der mehreren Atmungszyklen unter Verwendung des Satzes optischer Daten.

7. Am Körper tragbare Vorrichtung nach einem der Ansprüche 1-6, ferner umfassend:
ein Gehäuse; und
eine berührungsempfindliche Anzeige, die mit dem Gehäuse gekoppelt ist; wobei:
das Band dazu konfiguriert ist, das Gehäuse an dem Handgelenk eines Benutzers zu sichern; und
mindestens eine der Vielzahl von Elektroden auf dem Band positioniert ist.

8. Am Körper tragbare Vorrichtung (100, 200), umfassend:
Elektroden (106a, 106b, 210a, 210b), die zu Folgendem konfiguriert sind:
Anwenden eines oder mehrerer elektrischer Signale auf einen Benutzer; und
Erfassen des einen oder der mehreren elektrischen Signale von dem Benutzer;
einen Bewegungssensor (110), der mindestens teilweise innerhalb der am Körper tragbaren Vorrichtung positioniert ist; und
einen Prozessor (114), der zu Folgendem konfiguriert ist:
Durchführen einer oder mehrerer erster Impedanzmessungen unter Verwendung der Elektroden, um einen ersten Satz von Kontaktmetriken zu bestimmen;
Auswählen eines ersten Satzes der Elektroden unter Verwendung des ersten Satzes von Kontaktmetriken;
Durchführen einer oder mehrerer zweiter Impedanzmessungen unter Verwendung des ersten Satzes der Elektroden, um einen ersten Satz von Impedanzdaten zu erzeugen;
Bestimmen eines oder mehrerer Atmungszyklen des Benutzers aus dem Satz von Impedanzdaten;
Bestimmen eines Bewegungszustands des Benutzers unter Verwendung des Bewegungssensors;
als Reaktion darauf, dass der Bewegungszustand ein Bewegungskriterium erfüllt, Durchführen einer oder mehrerer dritter Impedanzmessungen unter Verwendung der Elektroden, um einen zweiten Satz von Kontaktmetriken zu bestimmen;
Auswählen eines zweiten Satzes der Elektroden unter Verwendung des zweiten Satzes von Kontaktmetriken; und
Durchführen einer oder mehrerer vierter Impedanzmessungen unter Verwendung des zweiten Satzes von Elektroden, um einen zweiten Satz von Impedanzdaten zu erzeugen.

9. Am Körper tragbare Vorrichtung nach Anspruch 8, wobei das Bestimmen des ersten und des zweiten Satzes von Kontaktmetriken Bestimmen einer Kontaktimpedanz für eine oder mehrere der Elektroden umfasst.

10. Am Körper tragbare Vorrichtung nach einem der Ansprüche 8-9, wobei das Auswählen des ersten und des zweiten Satzes von Elektroden Folgendes umfasst:
Auswählen eines ersten Teilsatzes der Elektroden zum Anwenden des einen oder der mehreren elektrischen Signale auf den Benutzer; und
Auswählen eines zweiten Teilsatzes der Elektroden zum Erfassen des einen oder der mehreren elektrischen Signale von dem Benutzer.

11. Am Körper tragbare Vorrichtung nach Anspruch 10, wobei der erste Teilsatz der Elektroden unterschiedliche Elektroden aus dem zweiten Teilsatz der Elektroden umfasst.

12. Am Körper tragbare Vorrichtung nach einem der Ansprüche 8-11, wobei der Prozessor zu Folgendem konfiguriert ist:
Bestimmen, ob der erste Satz von Kontaktmetriken einen Impedanzschwellenwert erfüllt; und
als Reaktion auf Bestimmen, dass der Satz von Kontaktmetriken den Impedanzschwellenwert erfüllt, Initiieren der einen oder der mehreren zweiten Impedanzmessungen.

13. Am Körper tragbare Vorrichtung nach Anspruch 12, wobei als Reaktion auf Bestimmen, dass der erste Satz von Kontaktmetriken den Impedanzschwellenwert nicht erfüllt, der Prozessor dazu konfiguriert ist, die eine oder die mehreren zweiten Impedanzmessungen abzubrechen.

14. Am Körper tragbare Vorrichtung nach einem der Ansprüche 8-13, wobei:
die Elektroden eine erste Gruppe von Elektroden, die an einer ersten Stelle der am Körper tragbaren Vorrichtung positioniert ist, und eine zweite Gruppe von Elektroden, die an einer zweiten Stelle der am Körper tragbaren Vorrichtung positioniert ist, umfassen; und
das Auswählen des ersten Satzes der Elektroden Auswählen eines des ersten Satzes von Elektroden oder des zweiten Satzes von Elektroden umfasst.

15. Am Körper tragbare Vorrichtung nach Anspruch 14, ferner umfassend:
ein Gehäuse; und
ein Band, das dazu konfiguriert ist, das Gehäuse an dem Benutzer zu sichern, wobei der erste Satz von Elektroden auf dem Band positioniert ist.

## Revendications

1. Dispositif portable (100, 200) comprenant :
un bracelet (207) configuré pour fixer le dispositif portable au poignet d'un utilisateur ;
une pluralité d'électrodes (106a, 106b, 210a, 210b) positionnées pour entrer en contact avec l'utilisateur lorsque le bracelet fixe le dispositif portable au poignet de l'utilisateur ;
un capteur de mouvement (110) positionné au moins partiellement à l'intérieur du dispositif portable ;
un processeur (114) configuré pour :
réaliser un premier ensemble de mesures en utilisant la pluralité d'électrodes pour déterminer des premières métriques de contact pour une pluralité de sous-ensembles de la pluralité d'électrodes ;
sélectionner un premier sous-ensemble de la pluralité de sous-ensembles en utilisant les premières métriques de contact ;
amener la pluralité d'électrodes à appliquer des signaux électriques à l'utilisateur ;
mesurer les signaux électriques provenant de l'utilisateur en utilisant le premier sous-ensemble sélectionné de la pluralité d'électrodes ;
générer un ensemble de données d'impédance à partir des signaux électriques mesurés ;
identifier un ou plusieurs cycles respiratoires en utilisant l'ensemble de données d'impédance ;
déterminer un état de mouvement de l'utilisateur en utilisant le capteur de mouvement ; et
en réponse à l'état de mouvement satisfaisant un premier critère de mouvement, réaliser un second ensemble de mesures en utilisant la pluralité d'électrodes pour déterminer des secondes métriques de contact pour une pluralité de sous-ensembles de la pluralité d'électrodes ;
sélectionner un second sous-ensemble de la pluralité de sous-ensembles en utilisant les secondes métriques de contact ; et
utiliser le second sous-ensemble sélectionné de la pluralité de sous-ensembles pour mesurer des signaux électriques provenant de l'utilisateur.

2. Dispositif portable selon la revendication 1, dans lequel chaque métrique de contact de la pluralité de métriques de contact représente une qualité de contact d'un sous-ensemble correspondant de la pluralité de sous-ensembles.

3. Dispositif portable selon l'une quelconque des revendications 1 à 2, dans lequel le processeur est configuré pour :
déterminer un premier sous-ensemble d'électrodes parmi la pluralité d'électrodes qui est utilisé pour appliquer les signaux électriques à l'utilisateur ; et
déterminer un second sous-ensemble d'électrodes parmi la pluralité d'électrodes qui est utilisé pour mesurer les signaux électriques provenant de l'utilisateur.

4. Dispositif portable selon la revendication 3, dans lequel le premier sous-ensemble d'électrodes comprend des électrodes différentes du second sous-ensemble d'électrodes.

5. Dispositif portable selon l'une quelconque des revendications 1 à 4, dans lequel le processeur est configuré pour mesurer des signaux électriques de l'utilisateur en réponse à la détermination que l'état de mouvement satisfait un critère de faible mouvement.

6. Dispositif portable selon l'une quelconque des revendications 1 à 5, comprenant en outre un capteur optique couplé au dispositif portable, dans lequel le processeur est configuré pour :
générer un ensemble de données optiques en utilisant le capteur optique ; et
identifier les un ou plusieurs cycles respiratoires en utilisant l'ensemble de données optiques.

7. Dispositif portable selon l'une quelconque des revendications 1 à 6, comprenant en outre :
un boîtier ; et
un écran tactile couplé au boîtier ; dans lequel :
le bracelet est configuré pour fixer le boîtier au poignet d'un utilisateur ; et
au moins une de la pluralité d'électrodes est positionnée sur le bracelet.

8. Dispositif portable (100, 200) comprenant :
des électrodes (106a, 106b, 210a, 210b) configurées pour :
appliquer un ou plusieurs signaux électriques à un utilisateur ; et
détecter les un ou plusieurs signaux électriques provenant de l'utilisateur ;
un capteur de mouvement (110) positionné au moins partiellement à l'intérieur du dispositif portable ; et
un processeur (114) configuré pour :
réaliser une ou plusieurs premières mesures d'impédance en utilisant les électrodes pour déterminer un premier ensemble de métriques de contact ;
sélectionner un premier ensemble des électrodes en utilisant le premier ensemble de métriques de contact ;
réaliser une ou plusieurs deuxièmes mesures d'impédance en utilisant le premier ensemble des électrodes pour générer un premier ensemble de données d'impédance ;
déterminer un ou plusieurs cycles respiratoires de l'utilisateur à partir de l'ensemble de données d'impédance ;
déterminer un état de mouvement de l'utilisateur en utilisant le capteur de mouvement ;
en réponse à l'état de mouvement satisfaisant un critère de mouvement, réaliser une ou plusieurs troisièmes mesures d'impédance en utilisant les électrodes pour déterminer un second ensemble de métriques de contact ;
sélectionner un second ensemble des électrodes en utilisant le second ensemble de métriques de contact ; et
réaliser une ou plusieurs quatrièmes mesures d'impédance en utilisant le second ensemble d'électrodes pour générer un second ensemble de données d'impédance.

9. Dispositif portable selon la revendication 8, dans lequel la détermination des premier et second ensembles de métriques de contact comprend la détermination d'une impédance de contact pour une ou plusieurs des électrodes.

10. Dispositif portable selon l'une quelconque des revendications 8 à 9, dans lequel la sélection des premier et second ensembles d'électrodes comprend :
la sélection d'un premier sous-ensemble des électrodes pour appliquer les un ou plusieurs signaux électriques à l'utilisateur ; et
la sélection d'un second sous-ensemble des électrodes pour détecter les un ou plusieurs signaux électriques provenant de l'utilisateur.

11. Dispositif portable selon la revendication 10, dans lequel le premier sous-ensemble des électrodes comprend des électrodes différentes du second sous-ensemble des électrodes.

12. Dispositif portable selon l'une quelconque des revendications 8 à 11, dans lequel le processeur est configuré pour :
déterminer si le premier ensemble de métriques de contact satisfait un seuil d'impédance ; et
en réponse à la détermination que l'ensemble de métriques de contact satisfait le seuil d'impédance, initier les une ou plusieurs deuxièmes mesures d'impédance.

13. Dispositif portable selon la revendication 12, dans lequel en réponse à la détermination que le premier ensemble de métriques de contact ne satisfait pas le seuil d'impédance, le processeur est configuré pour abandonner les une ou plusieurs deuxièmes mesures d'impédance.

14. Dispositif portable selon l'une quelconque des revendications 8 à 13, dans lequel :
les électrodes comprennent un premier groupe d'électrodes positionné au niveau d'un premier emplacement du dispositif portable et un second groupe d'électrodes positionné au niveau d'un second emplacement du dispositif portable ; et
la sélection du premier ensemble des électrodes comprend la sélection de l'un du premier ensemble d'électrodes ou du second ensemble d'électrodes.

15. Dispositif portable selon la revendication 14, comprenant en outre :
un boîtier ; et
un bracelet configuré pour fixer le boîtier à l'utilisateur, dans lequel le premier ensemble d'électrodes est positionné sur le bracelet.
